# EUROPEAN PATENT SPECIFICATION

(11) **EP 2 621 833 B1**
(45) Date of publication and mention of the grant of the patent: **09.11.2016**
(21) Application number: 11828443.9
(22) Date of filing: 28.09.2011
(51) Int. Cl.: B65D 85/16, A61F 13/15, A61F 13/472, B65B 11/10, B65D 65/40, B65D 75/42

(54) **CONTINUOUS PACKING PRODUCT OF ABSORBENT ARTICLES, EXTERIOR SHEETS, AND METHOD OF MANUFACTURING CONTINUOUS PACKING PRODUCT OF ABSORBENT ARTICLES**
PRODUKT ZUR KONTINUIERLICHEN VERPACKUNG VON SAUGFÄHIGEN ARTIKELN UND AUSSENHÜLLEN SOWIE VERFAHREN ZUR HERSTELLUNG DES PRODUKTS ZUR KONTINUIERLICHEN VERPACKUNG VON SAUGFÄHIGEN ARTIKELN UND AUSSENHÜLLEN
PRODUIT D'EMBALLAGE CONTINU D'ARTICLES ABSORBANTS, FEUILLES EXTÉRIEURES, ET PROCÉDÉ DE FABRICATION DE PRODUIT D'EMBALLAGE CONTINU D'ARTICLES ABSORBANTS

(30) Priority: 28.09.2010 JP 2010217429
(43) Date of publication of application: 07.08.2013
(73) Proprietor: Unicharm Corporation, Ehime 799-0111 (JP)
(72) Inventor: HARADA, Hiroyuki, Kanonji-shi Kagawa 769-1602 (JP); OKANO, Ryuji, Kanonji-shi Kagawa 769-1602 (JP); KOSAKO, Yusuke, Kanonji-shi Kagawa 769-1602 (JP); NISHITANI, Kazuya, Kanonji-shi Kagawa 769-1602 (JP)
(74) Representative: Dolleymores
(86) International application number: PCT/JP2011/005481
(87) International publication number: WO 2012/042877

(56) References cited:
- EP-A1- 0 573 664
- EP-A2- 1 300 125
- WO-A1-02/094678
- JP-A- 3 189 139
- JP-A- 9 154 880
- JP-A- 61 122 861
- JP-A- 2003 103 710
- JP-A- 2004 290 598
- JP-A- 2004 290 598
- JP-A- 2004 345 141
- JP-U- S 585 423
- US-A- 3 485 349
- US-A- 5 134 001
- US-A- 5 248 036
- US-A1- 2001 030 133

## Description

### Technical Field

The present invention relates to a continuous packing product, of absorbent articles, in which a continuous plurality of packing units which include the absorbent articles are provided.

### Background Art

In general absorbent articles such as sanitary napkins are sold in a manner in which a predetermined quantity of the absorbent articles are contained in a package which is formed by employing an exterior sheet such as a resin film.

In addition, it is widely known, mainly among developing countries that packing bodies for individually packing absorbent articles on one-by-one piece basis are sold in the manner of a continuous packing product in which a continuous plurality of packing units are provided in a longitudinal direction. A demander can purchase any required number of absorbent articles by separating a desired number of packing units from the continuous package of packing units. In addition, since the plurality of packing units that are continuous in the longitudinal direction can be sold while these packing bodies are hooked in an appropriate location in shop, it is convenient where there is limited space in a shopping facility. Patent Document 1 describes a continuous packing unit of absorbent articles in which packing units for individually housing the absorbent articles are continuous in plurality. Patent Document 2 describes a packing component in which a plurality of absorbent articles are packed with a packing member and wound in a roll. The packing member includes front and back cover sheets, at least one of which is formed continuously in a strip. The front and back cover sheets are sealed to house the absorbent articles. Patent Document 3 concerns a sheet for hermetic seal packaging, a compressed packaging bag and cellular cushioning material. The sheet comprises a thermoplastic resin sheet with a higher melting point coating.

### Citation List

### Patent Literature

[PTL 1] International Patent Application Publication No. 02/094678; [PTL 2] JP 2004 290598 A; [PTL 3] JP 2003 103710 A.

### Summary of Invention

### Technical Problem

However, conventional continuous packaging of absorbent articles has entailed the following problem. That is, since the continuous packing unit houses the absorbent articles therein on one-by-one piece basis, a large number of packing bodies are formed in comparison with packaging which contains some tens of absorbent articles. Therefore, an improvement in productivity of packing bodies is required.

For example, as such a method of manufacturing a continuous packaging of absorbent articles, the absorbent articles are continuously packed by means of exterior sheets, and then, the exterior sheets are thermally bonded to form a plurality of packing bodies which are continuous. In an attempt to improve productivity in such a manufacturing method, it is considered to reduce bonding time of the exterior sheet. However, if the bonding time is reduced, heat may not be sufficiently transmitted to the exterior sheet, and bonding faults may occur.

Therefore, it is an object of the present invention to provide a continuous packing product of absorbent articles, which are efficiently produced while preventing an occurrence of a fault in thermal bonding; a method of manufacturing a continuous packing product of absorbent articles which are adapted to manufacture the continuous packing unit of the absorbent articles; and exterior sheets for forming the continuous packing product of absorbent articles.

### Solution to Problem

According to the present invention in a first aspect there is provided a continuous packing product of absorbent articles as recited by claim 1.

According to the present invention in a second aspect, there is provided an exterior sheet provided with a partition region configured to pack continuously disposed absorbent articles and separately partition the absorbent articles by thermal bonding as recited by claim 5.

According to the present invention in a third aspect, there is provided a method of manufacturing a continuous packing product of absorbent articles as recited by claim 6.

### Advantageous Effects of Invention

According to characteristics of the present invention, there can be provided a continuous packing product of absorbent articles which are efficiently produced while preventing an occurrence of a fault in thermal bonding. That is, productivity of the continuous packing product of the absorbent articles can be improved.

### Brief Description of Drawings

[fig.1]Fig.1 is an entire perspective view of a continuous packing product of absorbent articles.
[fig.2]Fig.2 is a perspective view showing a packing unit.
[fig.3]Fig.3 is a sectional view of the packing unit taken along the line A-A of Fig. 2.
[fig.4]Fig.4 is a sectional view of the packing unit taken along the line B-B of Fig. 2.
[fig.5]Fig.5 is a sectional view of the packing unit taken along the line C-C of Fig. 2.
[fig.6]Fig.6 (a) to (e) are views showing a process for manufacturing a continuous packing product, according to the embodiment of the present invention.
[fig.7]Fig.7 (a) to (d) are views showing a process for manufacturing a continuous packing product, according to the embodiment of the present invention.
[fig.8]Fig.8 (a) to (d) are schematic sectional views of an exterior sheet.
[fig.9]Fig.9 is a sectional view of a packing unit according to an exemplary modification.

### Description of Embodiments

Next, a continuous packing product 1 of absorbent articles 10, according to the present invention, will be described with reference to the drawings. In the description of the drawings that follows, the same or similar constituent elements are designated by the same or similar reference numerals. However, it should be kept in mind that the drawings are merely schematic and rates of dimensions each are different from an actual one. Therefore, specific dimensions or the like should be de- termined in consideration of the following description. In addition, portions whose di- mensional interrelations or rates are different from each other can be included in the drawings as well.

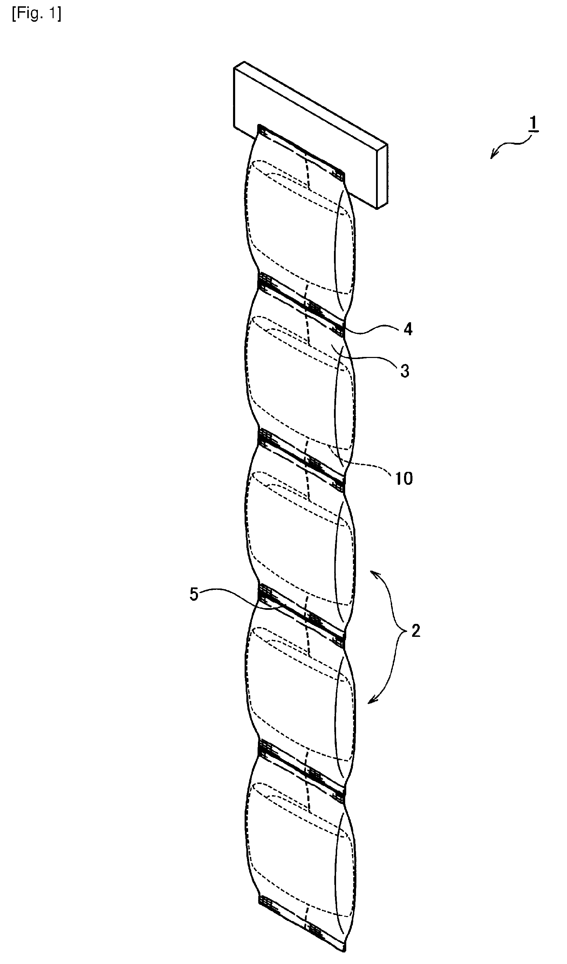

First, a schematic structure of a continuous packing product 1 of the absorbent articles 10 (hereinafter, referred to as the continuous packing product 1) will be described with reference to Fig. 1 to Fig. 5. Fig. 1 is an entire perspective view of the continuous packing product 1. As shown in Fig. 1, the continuous packing product 1 is formed by absorbent articles 10 which are sanitary napkins and exterior sheets 3 for wrapping the absorbent articles 10. The exterior sheets 3 are formed in a continuous sheet shape, and are adapted to pack the absorbent articles 10 in the shape of a bag.

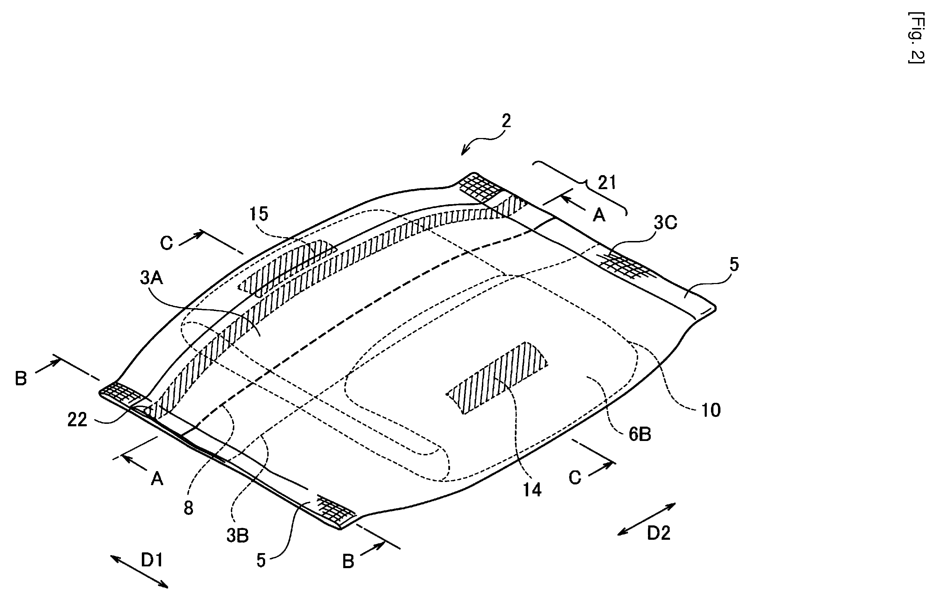

Partition units 5 for partitioning the absorbent articles that are wrapped by the exterior sheets 3 on a one-by-one piece basis are formed at the continuous packing product 1. Each of the partition units 5 is formed by a bonded region 3C of the exterior sheet 3 (refer to Fig. 2) being bonded by means of thermal bonding (deposition). By means of the partition units 5, a plurality of packing bodies 2 for packing the absorbent articles 10 on one-by-one piece basis are continuously formed. In addition, five packing bodies 2 are continuous, and one continuous packing product 1 is formed.

In addition, a cutoff line unit 4 to which perforation processing is applied is formed at the partition unit 5, and packing bodies 2 can be separated from the continuous packing product 1 according to a required number of absorbent articles 10.

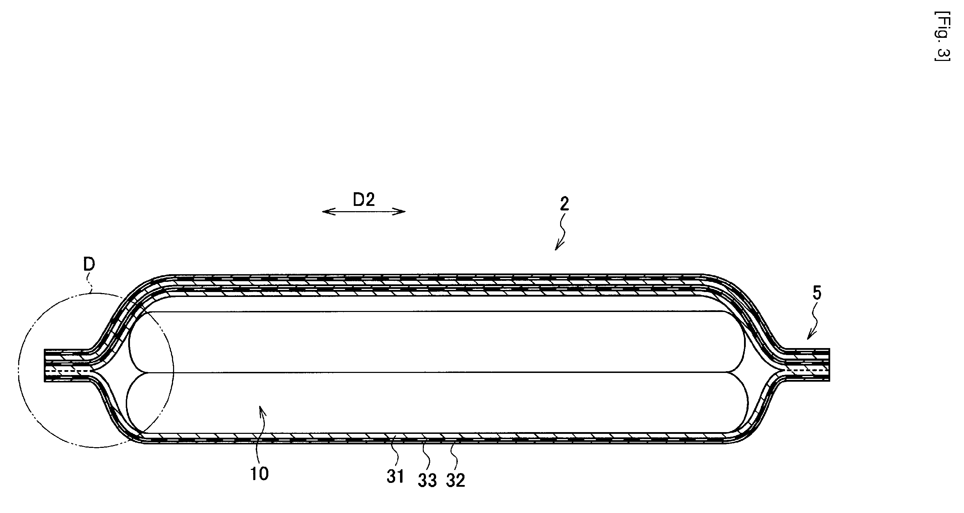

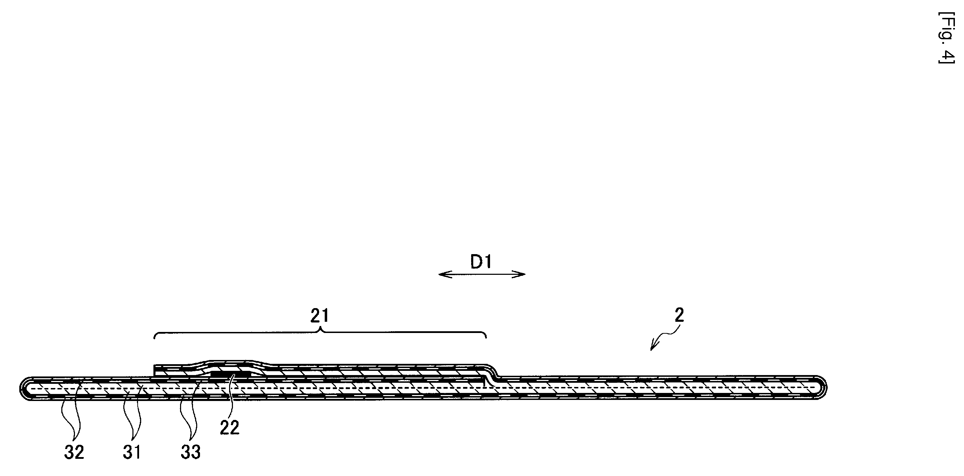

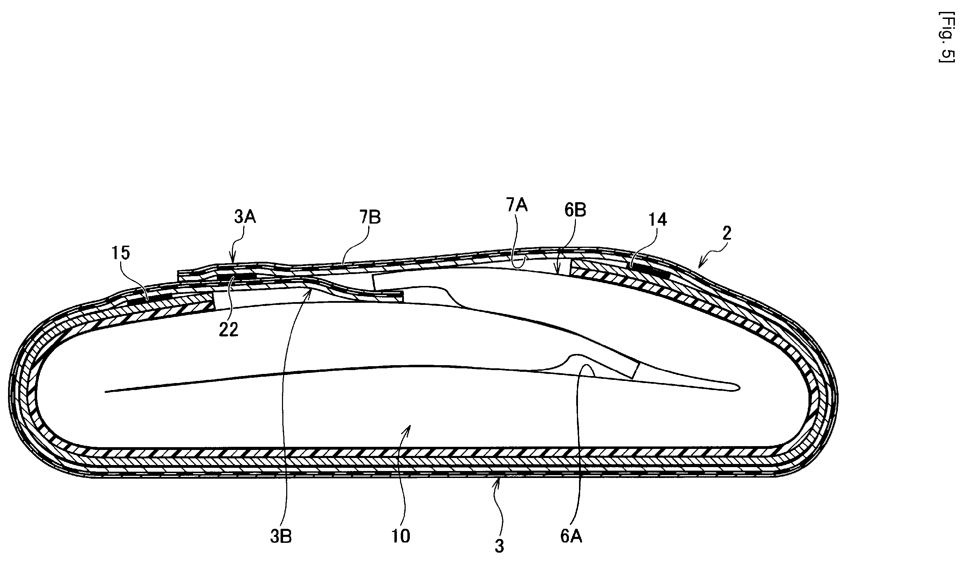

Fig. 2 is a perspective view showing a packing unit 2 which is separated from the continuous packing product 1. Fig. 3 is a sectional view of the packing unit 2 taken along the line A-A of Fig. 2. Fig. 4 is a sectional view of the packing unit 2 taken along the line B-B of Fig. 2. Fig. 5 is a sectional view of the packing unit taken along the line C-C of Fig. 2.

Fig. 3 is a sectional view of a packing unit 2 in a continuous direction D2 in which the packing bodies 2 extend continuously; Fig. 4 is a sectional view of a partition region 5 of the packing unit 2. The partition region 5 is provided at the bonded region 3C of the exterior sheet 3. A partition region 5 on which the exterior sheet 3 is deposited is at each end part of the packing body 2 in Fig. 3. The exterior sheet 3 has a first resin layer 31, a second resin layer 32, and a print layer 33. The first resin layer 31 is formed of a thermoplastic resin with a comparatively low melting point (relative to the second layer 32), which is melted by means of thermal bonding. Polyethylene can be employed, for example, as a material for the first resin layer 31, and further preferably, a straight chain-like low density polyethylene (LLDPE) and a low density polyethylene (LDPE) can be employed.

Since polyethylene is comparatively low in melting point among thermoplastic resins (for example, in comparison with polypropylene), it is possible for resin layer 31 to have a difference in melting point to a resin forming the second resin layer 32. Therefore, when thermal bonding is done, only the first resin layer 31 can be melted without melting the second resin layer 32. In addition, since polyethylene UV-resistance (ultraviolet-ray resistance), it is possible to prevent degradation due to the UV of the absorbent articles that are housed therein.

The second resin layer 32 is a thermoplastic resin having a higher melting point than that of the thermoplastic resin forming the first resin layer, and may be formed of a resin which is soluble in solvent. The second resin layer is formed of a thermoplastic resin which is insoluble by means of thermal bonding, and is adapted to thermally resist and protect the first resin layer 31 without being melted at the time of thermal bonding. Polyamide such as nylon or imide can be exemplified as a material for the second resin layer 32.

Any solvent may be employed as long as it melts the thermoplastic resin forming the second resin layer 32, and a hydrocarbon having its boiling point of 150 degrees or less can be exemplified. Solvents include, but are not limited to, hexane, xylene, toluene, ethyl acetate, butyl acetate, isopropanol, butanol, and methyl ethyl ketone.

The print layer 33 is disposed between the first resin layer 31 and the second resin layer 32. A method of forming the print layer is not limited. For example, the print layer 33 may be formed by means of a printing method such as flexographic printing, a sink printing or the like, or alternatively, may be formed by blowing ink through spraying. In addition, by providing the print layer 33, visible light can be hardly transmitted, and then, the absorbent articles 10 that are housed therein can be hardly visually recognized from the outside of the exterior sheets 3.

The exterior sheet 3 is disposed so that the second resin layer 32 configures an outside face 7B of the exterior sheet 3 and the first resin layer 31 configures an inside face 7A of the exterior sheet 3. Thermal bonding is done from the outside face side of the exterior sheet 3, whereby the first resin layer 31 is melted, the exterior sheet 3 of a bonding region 3C is bonded therewith, and the partition region 5 is formed.

In addition, as shown in Fig. 2 and Fig. 5, the packing unit 2 may be formed with the absorbent article 10 and the exterior sheet 3 folded together in a state in which a back face 6B which is a cloth (clothing) contact surface of the absorbent article 10 and the inside face 7Af the exterior sheet 3 are opposite to each other.

Specifically, the exterior sheet 3 is disposed at the side of the back face 6B of the absorbent article 10, and then, at least a part of the back face 6B of the absorbent article 10 is fixed to the inside face 7A of the exterior sheet 3. The back face 6B of the absorbent article 10 and the inside face 7A of the exterior sheet 3 are fixed to each other via an adhesive unit 14 and an adhesive unit 15. The absorbent article 10 and the exterior sheet 3 that are fixed to each other by means of the adhesive unit 14 and the adhesive unit 15 are folded into three together along a folding direction D1 toward the side of the surface 6A which is a skin contact surface of the absorbent article 10.

The absorbent article 10 and the exterior sheet 3 are folded together, whereby an overlap region 21 (refer to Fig. 2) in which an end part 3A and an end part 3B in the folding direction D1 of the exterior sheet 3 are superimposed on each other is formed. At the packing unit 2, an opening cutoff line part 8 is formed along a continuous direction D2 in which the packing units 2 are continuous. The opening cutoff line part 8 is cut off, whereby the packing unit 2 is released, and then, the absorbent article that is housed therein is exposed.

A bonding region 22 (refer to Fig. 2) in which the end part 3A and the end part 3B of the exterior sheet 3 are bonded with each other is formed at a part of the overlap region 21. The bonding region 22 is formed by being adhesively bonded by means of thermal bonding, a biting emboss, an adhesive agent, an adhesive tape or the like.

The partition region 5 that corresponds to the bonded region 3C of the exterior sheet 3 is at each end part of the packing bodies 2 in a continuous direction D2 in which the packing bodies 2 are continuous. Since the partition region 5 is adhesively bonded by means of thermal bonding of the exterior sheet 3, the absorbent article 10 is established in a sealed sanitary state.

Next, a method of manufacturing a continuous packing product 1 will be described. Herein, in particular, a method of manufacturing exterior sheets 3 and a continuous packing product 1 will be mainly described. Other portions such as the absorbent articles 10 can be manufactured in accordance with well known manufacturing methods.

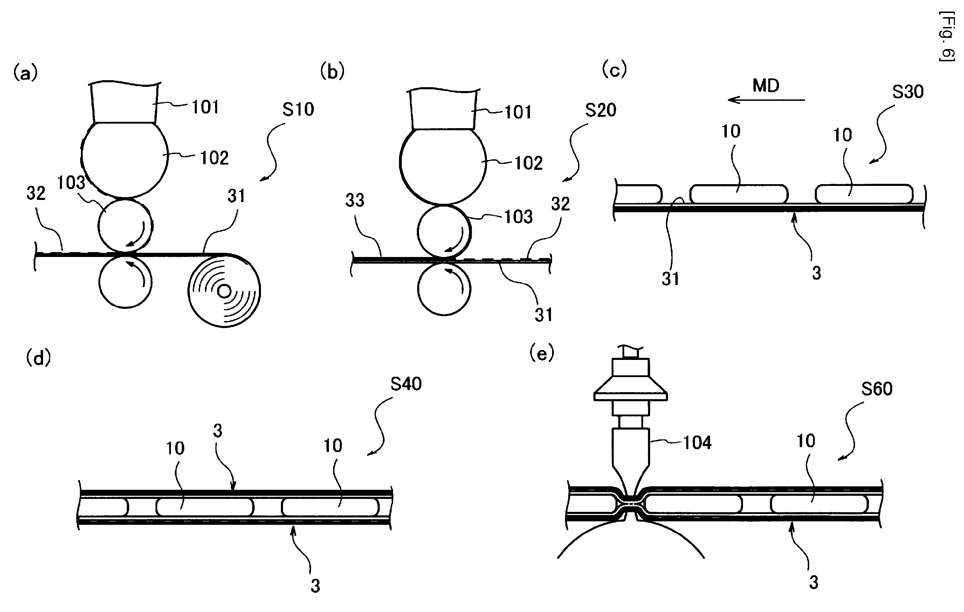

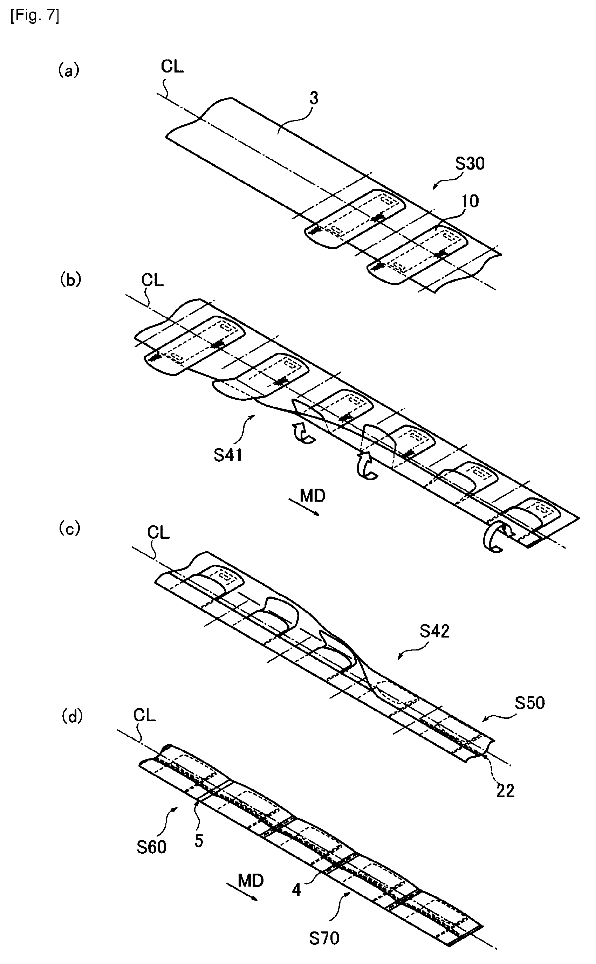

Fig. 6 and Fig. 7 are views schematically showing a process of manufacturing a continuous packing product 1. Fig. 6 shows a cross section taken along a conveyance direction MD of absorbent articles 10; and Fig. 7 is a perspective view of the absorbent article 10 and the exterior sheet 3. As shown in Fig. 6 and Fig. 7, the process of manufacturing a continuous packing product 10 includes: a print layer forming step S10; a second resin layer forming step S20; an absorbent article placement step S30; a folding step S40; an exterior sheet bonding step S50; a packing body forming step S60; and a cutoff line part forming step S70. The print layer forming step S10 and the second resin layer forming step S20 are included in the process of manufacturing exterior sheets 3. In addition, the folding step S40, as shown in Fig. 6, includes a first folding step S41 and a second folding step S42.

In the print layer forming step S10, as shown in Fig. 6 (a), gravure printing is applied to the first resin layer 31 made of a resin film, and then, the print layer 33 is formed. An applying device for applying gravure printing is provided with: a reservoir unit 101 in which ink is reserved; a transfer roll 102 to which ink is transferred from the reservoir unit 101; and an applying roll 103 to which ink is transferred from the transfer roll 102. Ink is applied to the first resin layer 31 by means of the applying roll 103.

In the second resin layer forming step S20, as shown in Fig. 6 (b), the second resin layer 32 is formed on the print layer 33. Specifically, a solution obtained by melting a nylon, which is a thermoplastic resin with higher melting point than that of the resin forming the first resin layer 31, in hexane as a solvent, is applied onto the print layer 33. The applying device for applying a solution is similar to that in the print layer forming step S10, and therefore, a duplicate description is omitted. In this manner, since the print layer forming step S10 and the second resin layer forming step S20 can be carried out by means of the applying device having the similar structure, both of these steps can be carried out in the same facility, and productivity can be improved. In addition, the exterior sheets 3 are manufactured by means of the print layer forming step S10 and the second resin layer forming step S20.

In the absorbent article placement process S30, as shown in Fig. 6 (c) and Fig. 7 (a), the absorbent articles 10 are fixed at predetermined intervals on the exterior sheets 3 which are continuous along the conveyance direction MD. At this time, the exterior sheet 3, the first resin layer 31, and the back face of the absorbent article 10 are opposite to each other and are disposed so that a lengthwise direction of the absorbent article 10 is orthogonal to the conveyance direction MD.

A folding step S40 has the first folding step S41 shown in Fig. 7 (b) and the second folding step S42 shown in Fig. 7 (c). In the first folding step S41, the exterior sheet 3 and the absorbent article 10 are lifted from one end side in the folding direction D1 that is orthogonal to the conveyance direction MD, and then, are folded together toward a centerline CL in the folding direction D1. A folding line of the exterior sheet 3 and the absorbent article 10 that have been folded is taken along the continuous direction D2 that is parallel to the conveyance direction MD.

In the second folding step S42, the exterior sheet 3 and the absorbent article 10 are lifted from the opposite side from that of the first folding step S41, and then, are folded together toward the centerline CL in the folding direction D1. By means of this step, the overlap region 21 (Fig. 2) is formed at continuous packing bodies 2. Fig. 6 (d) shows the exterior sheet 3 and the absorbent article 10 which are folded by means of the folding step S40.

In the exterior sheet bonding step S50, as shown in Fig. 7 (c), a bonding region 22 is bonded by means of an adhesive agent in an overlap region 21 which is formed when both end parts 3A and 3B in the folding direction D1 of the exterior sheet 3 are folded on each other.

In the packing product forming step S60, as shown in Fig. 6 (e) and Fig. 7 (d), the bonding region 3C of the exterior sheet 3 is thermally bonded, whereby adhesive bonding treatment to partitioning the absorbent articles 10 on a one-by-one piece basis is made. By means of this step, packing units 2 for packing the absorbent articles 10 on a one-by-one piece basis are formed.

In a cutoff line part forming step S70, perforation forming process is performed at the partition unit 5. By the perforation forming process, a cutoff line part 4 which is capable of individually cutting off the packing body 2 can be formed. The steps S10 to S70 described above are executed, whereby a continuous packing product 1 is completed.

As has been described above, according to the continuous packing product 1, since the second resin layer 32 is formed by dissolving the thermoplastic resin forming the second resin layer 32 in a solvent and then applying the melted resin, the second resin layer 32 can be thinly formed in comparison with the first resin layer 31 which is a resin film. Therefore, heat of thermal bonding is easily transmitted to the first resin layer 31, a heating time of thermal bonding is reduced, and productivity can be improved. Further, since transmission efficiency of the heat to the first resin layer is improved without increasing the heating temperature of thermal bonding, it is possible to prevent melting of the second resin layer 32 constituting the outside face 7B of the exterior sheet 3 due to a rise of the heating temperature and then prevent the melted resin from adhering to a bonding jig.

As an example, melting points of the straight chain-like low-density polyethylene and low density polyethylene which form the first resin layer 31 are about 110 degrees Celsius, and a melting point of nylon forming the second resin layer 32 is about 160 degrees Celsius to 170 degrees Celsius, and since a difference in the melting points are 50 degrees Celsius or more, only the first resin layer 31 can be melted without melting the second resin layer 32 in a thermal bonding, and the sealing property of the first resin layer 31 can be maintained.

It should not be understood that the discussion and drawings forming a part of this disclosure limit the present invention. From this disclosure, a variety of alternative embodiments, examples, and operational techniques would be self-evident to one skilled in the art within the scope of the claims.

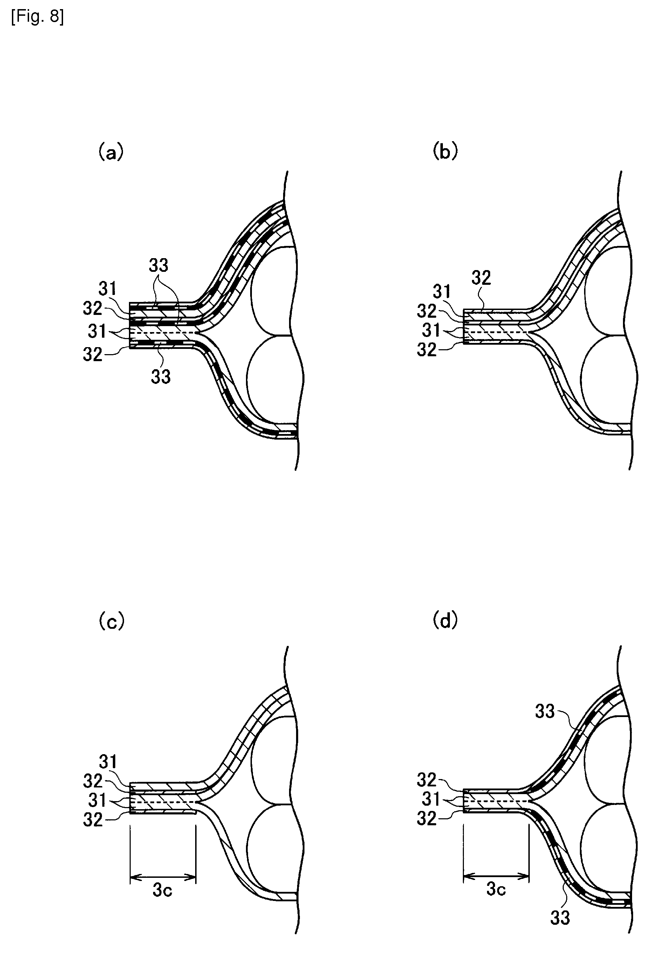

Exterior sheets according to exemplary modifications and the present invention will be described with reference to Fig. 8. Figs. 8 (a) to (c) are a sectional view of the exterior sheet 3 according to exemplary arrangements Fig. 8 (d) is a sectional view of an exterior sheet 3 according to the present invention. Fig. 8 (a) is an enlarged view showing a portion D shown in Fig. 3 of the exterior sheet according to the arrangement above, and Figs. 8 (b) to (d) are enlarged sectional views of portions corresponding to the portion Din the exterior sheet 3 according to modifications. Like constituent elements of the exterior sheet 3 are designated by like reference numerals, and a duplicate description is omitted.

As shown in Fig. 8 (a), on the exterior sheet 3 according to an example, the print layer 33 is laminated in a full region of the first resin layer 31, and the second resin layer 32 is laminated in a full region of the print layer 33. In this way, since the print layer 33 is laminated in the full region of the first resin layer 31, a decorative effect due to characters or patters and the like of the print layer 33 can be attained in the full region of the exterior sheet 3.

Further, the second resin layer 32 is laminated in the full region of the print layer 33, whereby the print layer 33 can be protected by the second resin layer 32. In order to visually recognize the print layer 33 from the outside of the packing unit 2, it is desirable to configure the print layer 33 so as to be visually discemable from the side of the second resin layer 32 forming the outside face 7B of the exterior sheet 3. Specifically, the second resin layer 32 may be formed of a transparent or semitransparent material.

An exterior sheet 3, as shown in the example of Fig. 8 (b), may be configured to have the first resin layer 31 and the second resin layer 32 without comprising the print layer 33. With such a structure, since the step of forming print layer 33 is eliminated, productivity can be further improved, and reduction of production time and cost can be attained.

In an exterior sheet 3, as shown in the example of Fig. 8 (c), the second resin layer 32 may be formed in only the bonded (partition) region 3C. In the region 3C, heat resistance processing is made by means of the second resin layer 32, whereby, when thermal bonding is applied from the side of the second resin layer 32, the first resin layer 31 can be protected by means of the second resin layer 32.

While, in the exemplary modification shown in Fig. 8 (c), the exterior sheet 3 is formed by means of the first resin layer 31 and the second resin layer 32, a print layer may be formed between the first resin layer 31 and the second resin layer 32, or alternatively, a print layer may be formed in the full region of the first resin layer 31.

In the arrangement of Fig. 8 (d) in accordance with the present invention, the print layers 33 are intermittently formed on the exterior sheet 3 and the print layers 33 need not be formed in the bonded region 3C. In this way, the print layers 33 need not be formed in the bonded region 3C, whereby the heat from the bonding jig can be easily transmitted to the first resin layer 31.

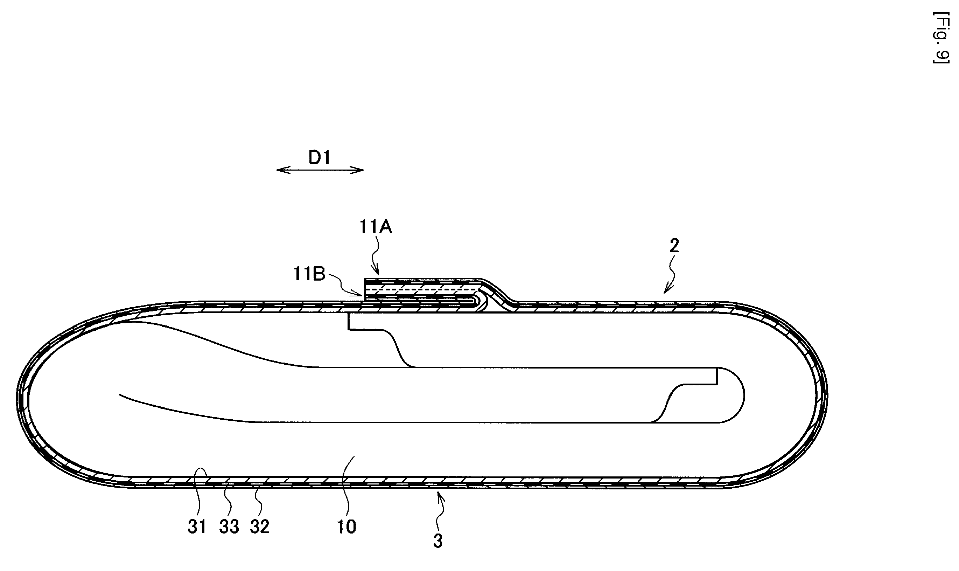

Next, a packing unit according to an exemplary modification will be described with reference to Fig. 9. Fig. 9 is a sectional view of the folding direction D1 that is orthogonal to the continuous direction of the packing bodies 2 according to the exemplary modification. The exterior sheets 3 of the packing bodies 2 according to the exemplary modification are bonded with each other by means of thermal bonding while the first resin layer 31 and the first resin layer 31 are opposite to each other at both end parts 11A and 11B in the folding direction D1. In this way, these resin layers may be bonded with each other by means of thermal bonding, not only at the partition units 5 for partitioning the packing body 2 and the packing body 2 but also in a bonding region 22 in which both end pats in the folding direction D1 of the exterior sheet 3 is to be bonded as well.

In addition, while, in the embodiment of the present invention, one absorbent article was housed in a packing unit, two or more absorbent articles may be configured so as to be housed in a packing unit.

Further, the absorbent article of the present invention may be a panty liner (a vaginal discharge sheet), an incontinent pad or the like other than the above absorbent article.

Thus, it is a mater f course that the present invention encompasses a variety of embodiments or the like, which are not described herein. Therefore, technical scope of the present invention is defined only by the specific matters of the invention according to the claims that are reasonable from the foregoing description.

Embodiments, and variations of any aspect of the invention, may be such that an absorbent article and exterior sheet in a packing unit are folded in at least a first direction which is orthogonal to the continuous direction of the packing product. Embodiments may be such that the absorbent article and exterior sheet are also folded in the opposite direction to the first orthogonal direction to form an overlap region.

A method of manufacturing a continuous package may, in any aspect, include a step of folding the exterior sheet and the absorbent article along a direction which is orthogonal to a continuous direction in which the absorbent articles are continuous, and then, wrapping the absorbent article by means of the exterior sheet.

They may further include a step which the exterior sheet and absorbent article are also folded in a second orthogonal direction opposite to the first orthogonal direction to form an overlap portion.

The above features and steps may be used in any embodiments or aspects of the invention.

## Claims

1. A continuous packing product (1) of absorbent articles (10) in which a plurality of packing units (2) are continuous, with one or more absorbent articles being packed in each unit by means of an exterior sheet (3) comprising a thermally bonded product partition region (3C) configured to partition the packing product into packing units; **characterised in that**
the exterior sheet has:
a first resin layer (31) which is formed of a thermoplastic resin; and
a second resin layer (32) which is formed of a thermoplastic resin with a melting point higher than that of the thermoplastic resin forming the first resin layer;
the second resin layer is laminated on the first resin layer in at least the bonded region;
the thermoplastic resin of the second resin layer is soluble in a solvent;
a print layer (33) to which printing is applied is formed at a side of the first resin layer facing the second resin layer; and
the print layer is intermittently formed and is not laminated on the first resin layer in the
bonded region.

2. The continuous packing product of absorbent articles, according to claim 1, wherein:
the packing units individually contain the absorbent articles.

3. The continuous packing product of absorbent articles, according to claim 1 or claim 2, wherein:
the thermoplastic resin forming the first resin layer is polyethylene; and the
thermoplastic resin forming the second resin layer is polyamide.

4. The continuous packing product of absorbent articles, according to any one of claims 1 to 3, wherein:
the print layer is formed between the first resin layer and the second resin layer, and is
visually discernable from an outside of the second resin layer.

5. An exterior sheet (3) provided with a partition region (5) configured to pack continuously disposed absorbent articles (10) and separately partition the absorbent articles by means of thermal bonding, **characterised in that**:
the exterior sheet has: a first resin layer (31) which is formed of a thermoplastic resin;
and a second resin layer (32) which is formed of a thermoplastic resin with a melting point higher than that of the thermoplastic resin forming the first resin layer;
the second resin layer is laminated on the first resin layer in at least the partition region;
the thermoplastic resin configuring the second resin layer is soluble in a solvent; and
a print layer (33) to which printing is applied is formed at a side of the first resin layer facing the second resin layer; and
the print layer is intermittently formed and is not laminated on the first resin layer in the
bonded region.

6. A method of manufacturing a continuous packing product (1) of absorbent articles (10), **characterised by**:
a first step of forming an exterior sheet (3) by providing a resin film formed of a thermoplastic resin, forming a print layer (33) to which printing is applied on the resin film, and applying to the resin film a solution which is obtained by dissolving in a solvent a thermoplastic resin with a melting point which is higher than that of the thermoplastic resin forming the resin film, the print layer being formed on a surface of the resin film facing the solution;
a second step of continuously disposing absorbent articles each on the resin film of the exterior sheet;
a step of folding the exterior sheet and the absorbent article along a direction which is orthogonal to a continuous direction in which the absorbent articles are continuous, and then, wrapping the absorbent article by means of the exterior sheet; and
a fourth step of partitioning the exterior sheet having the absorbent article wrapped thereby, by means of thermal bonding, and then, continuously forming a plurality of packing units (2) by which the absorbent articles are packed,
wherein the print layer is intermittently formed and is not laminated on the first resin layer in the bonded region.

## Patentansprüche

1. Kontinuierliches Verpackungsprodukt (1) von absorbierenden Artikeln (10), in dem eine Vielzahl von Verpackungseinheiten (2) kontinuierlich mit einem oder mehreren absorbierenden Artikeln vorliegt, die in jeder Einheit mithilfe einer äußeren Lage (3) verpackt sind, die einen thermisch verbundenen Produktaufteilungsbereich (3C) umfasst, der zur Aufteilung des Verpackungsprodukts in Verpackungseinheiten konfiguriert ist;
**dadurch gekennzeichnet, dass**
die äußere Lage Folgendes aufweist:
eine erste Harzschicht (31), die aus einem thermoplastischen Harz gebildet ist; und
eine zweite Harzschicht (32), die aus einem thermoplastischen Harz mit einem Schmelzpunkt gebildet ist, der höher ist als der des thermoplastischen Harzes, das die erste Harzschicht bildet;
die zweite Harzschicht auf die erste Harzschicht in zumindest dem verbundenen Bereich laminiert ist;
das thermoplastische Harz der zweiten Harzschicht in einem Lösungsmitteln löslich ist;
eine Druckschicht (33), auf die ein Bedruck aufgetragen ist, auf einer Seite der ersten Harzschicht ausgebildet ist, die der zweiten Harzschicht gegenüberliegt; und
die Druckschicht intermittierend ausgebildet ist und nicht auf die erste Harzschicht in dem verbundenen Bereich laminiert ist.

2. Kontinuierliches Verpackungsprodukt von absorbierenden Artikeln nach Anspruch 1, wobei die Verpackungseinheiten die absorbierenden Artikel einzeln enthalten.

3. Kontinuierliches Verpackungsprodukt von absorbierenden Artikeln nach Anspruch 1 oder Anspruch 2, wobei:
das thermoplastische Harz, das die erste Harzschicht bildet,
Polyethylen ist; und das thermoplastische Harz, das die zweite Harzschicht bildet, Polyamid ist.

4. Kontinuierliches Verpackungsprodukt von absorbierenden Artikeln nach einem der Ansprüche 1 bis 3, wobei:
die Druckschicht zwischen der ersten Harzschicht und der zweiten Harzschicht ausgebildet ist und visuell von einer Außenseite der zweiten Harzschicht erkennbar ist.

5. Äußere Lage (3), die mit einem Aufteilungsbereich (5) bereitgestellt ist, der zur Verpackung von kontinuierlich angeordneten absorbierenden Artikeln (10) und zur abgetrennten Aufteilung der absorbierenden Artikel mithilfe von thermischer Verbindung konfiguriert ist, **dadurch gekennzeichnet, dass**:
die äußere Lage Folgendes aufweist: eine erste Harzschicht (31), die aus einem thermoplastischen Harz gebildet ist; und eine zweite Harzschicht (32), die aus einem thermoplastischen Harz mit einem Schmelzpunkt gebildet ist, der höher ist als der des thermoplastischen Harzes, das die erste Harzschicht bildet;
die zweite Harzschicht auf die erste Harzschicht in zumindest dem Aufteilungsbereich laminiert ist;
das thermoplastische Harz, das die zweite Harzschicht konfiguriert, in einem Lösungsmitteln löslich ist; und
eine Druckschicht (33), auf die ein Bedruck aufgetragen ist, auf einer Seite der ersten Harzschicht ausgebildet ist, die der zweiten Harzschicht gegenüberliegt; und
die Druckschicht intermittierend ausgebildet ist und nicht auf die erste Harzschicht in dem verbundenen Bereich laminiert ist.

6. Verfahren zur Herstellung eines kontinuierlichen Verpackungsprodukts (1) von absorbierenden Artikeln (10), **gekennzeichnet durch**:
einen ersten Schritt der Bildung einer äußeren Lage (3) **durch** Bereitstellen einer Harzfolie, die aus einem thermoplastischen Harz gebildet ist, Bilden einer Druckschicht (33), auf die ein Bedruck aufgetragen ist, auf der Harzfolie und Auftragen einer Lösung auf die Harzfolie, die **durch** Lösen eines thermoplastischen Harzes mit einem Schmelzpunkt, der höher ist als der des thermoplastischen Harzes, das die Harzfolie bildet, in einem Lösungsmittel erhalten wird, wobei die Druckschicht auf einer Oberfläche der Harzfolie ausgebildet ist, die der Lösung gegenüberliegt;
einen zweiten Schritt der kontinuierlichen Anordnung von absorbierenden Artikeln jeweils auf der Harzfolie der äußeren Lage;
einen Schritt der Faltung der äußeren Lage und des absorbierenden Artikels entlang einer Richtung, die senkrecht zu einer kontinuierlichen Richtung liegt, in der die absorbierenden Artikel kontinuierlich vorliegen, und dann Umhüllung des absorbierenden Artikels mithilfe der äußeren Lage; und
einen vierten Schritt der Aufteilung der äußeren Lage mit dem absorbierenden Artikel, der **dadurch** umhüllt ist, mithilfe von thermischem Verbinden und dann der kontinuierlichen Bildung einer Vielzahl von Verpackungseinheiten (2), **durch** die die absorbierenden Artikel verpackt sind,
wobei die Druckschicht intermittierend ausgebildet ist und nicht auf die erste Harzschicht in dem verbundenen Bereich laminiert ist.

## Revendications

1. Produit d'emballage continu (1) d'articles absorbants (10) dans lequel une pluralité d'unités d'emballage (2) sont continues, avec un ou plusieurs articles absorbants qui sont emballés dans chaque unité au moyen d'une feuille extérieure (3) comportant une région de division de produits thermiquement liée (3C) configurée à des fins de division du produit d'emballage en unités d'emballage ;
**caractérisé en ce que**
la feuille extérieure a :
une première couche de résine (31) qui est formée à partir d'une résine thermoplastique ; et
une deuxième couche de résine (32) qui est formée à partir d'une résine thermoplastique ayant un point de fusion supérieur à celui de la résine thermoplastique formant la première couche de résine ;
la deuxième couche de résine est stratifiée sur la première couche de résine dans au moins la région liée ;
la résine thermoplastique de la deuxième couche de résine est soluble dans un solvant ;
une couche d'impression (33) sur laquelle une impression est appliquée est formée au niveau d'un côté de la première couche de résine qui fait face à la deuxième couche de résine ; et
la couche d'impression est formée de manière intermittente et n'est pas stratifiée sur la première couche de résine dans la région liée.

2. Produit d'emballage continu d'articles absorbants selon la revendication 1, dans lequel :
les unités d'emballage contiennent individuellement les articles absorbants.

3. Produit d'emballage continu d'articles absorbants selon la revendication 1 ou la revendication 2, dans lequel :
la résine thermoplastique formant la première couche de résine est du polyéthylène ;
et la résine thermoplastique formant la deuxième couche de résine est du polyamide.

4. Produit d'emballage continu d'articles absorbants selon l'une quelconque des revendications 1 à 3, dans lequel :
la couche d'impression est formée entre la première couche de résine et la deuxième couche de résine, et est visuellement discernable depuis un extérieur de la deuxième couche de résine.

5. Feuille extérieure (3) mise en oeuvre avec une région de division (5) configurée à des fins d'emballage d'articles absorbants (10) disposés en continu et à des fins de division des articles absorbants de manière séparée au moyen d'une liaison thermique, **caractérisée en ce que** :
la feuille extérieure a : une première couche de résine (31) qui est formée à partir d'une résine thermoplastique ; et une deuxième couche de résine (32) qui est formée à partir d'une résine thermoplastique ayant un point de fusion supérieur à celui de la résine thermoplastique formant la première couche de résine ;
la deuxième couche de résine est stratifiée sur la première couche de résine dans au moins la région de division ;
la résine thermoplastique configurant la deuxième couche de résine est soluble dans un solvant ; et
une couche d'impression (33) sur laquelle une impression est appliquée est formée au niveau d'un côté de la première couche de résine qui fait face à la deuxième couche de résine ; et
la couche d'impression est formée de manière intermittente et n'est pas stratifiée sur la première couche de résine dans la région liée.

6. Procédé de fabrication d'un produit d'emballage continu (1) d'articles absorbants (10), **caractérisé par** :
une première étape consistant à former une feuille extérieure (3) par la mise en oeuvre d'un film de résine formé à partir d'une résine thermoplastique, former une couche d'impression (33) sur laquelle une impression est appliquée sur le film de résine, et appliquer sur le film de résine une solution qui est obtenue par dissolution dans un solvant d'une résine thermoplastique ayant un point de fusion qui est supérieur à celui de la résine thermoplastique formant le film de résine, la couche d'impression étant formée sur une surface du film de résine qui fait face à la solution ;
une deuxième étape consistant à disposer en continu des articles absorbants chacun sur le film de résine de la feuille extérieure ;
une étape consistant à plier la feuille extérieure et l'article absorbant le long d'une direction qui est perpendiculaire par rapport à une direction continue dans laquelle les articles absorbants sont continus, et puis, envelopper l'article absorbant au moyen de la feuille extérieure ; et
une quatrième étape consistant à diviser la feuille extérieure ayant l'article absorbant enveloppé par celle-ci, au moyen d'une liaison thermique, et puis, former en continu une pluralité d'unités d'emballage (2) au moyen desquelles les articles absorbants sont emballés,
dans lequel la couche d'impression est formée de manière intermittente et n'est pas stratifiée sur la première couche de résine dans la région liée.
